# EUROPEAN PATENT APPLICATION

(11) **EP 4 545 061 A1**
(43) Date of publication of application: **30.04.2025**
(21) Application number: 23205619.2
(22) Date of filing: 24.10.2023
(51) Int. Cl.: A61K 8/04, A61K 8/27, A61K 8/29, A61K 8/34, A61Q 1/02, A61Q 17/04

(54) **STABLE DISPERSIONS OF LIPOPHOBIC INGREDIENTS IN POWDER FORM**

(71) Applicant: Oleon N.V., 9940 Ertvelde (BE)
(72) Inventor: PEETERS, Hilde, 3140 KEERBERGEN (BE); PUPPETT, Mauro, 60350 Vieux Moulin (FR)
(74) Representative: Santarelli

(57) **Abstract**

The present invention relates to stable dispersions of lipophobic ingredients in powder form and products comprising them.

## Description

The present invention relates to the use of a specific compound as a dispersing agent of lipophobic ingredients in powder form.

The lipophobic ingredient is preferably a mineral pigment, a mineral ultraviolet (UV) filter, an organic dye, and /or a mineral texturing agent, all of them being in powder form.

A pigment is a natural or synthetic substance that is intended for coloring and/or opacifying.

Pigments are typically provided as dry powders, largely consisting of aggregates with a small percentage of primary particles.

Aggregates are formed by primary particles of pigment being tightly cemented together by pressure and heat during the pigment production process. Although they are relatively small, they are difficult to break apart. The dispersion of those larger aggregates is more difficult.

Mineral ultraviolet (UV) filters absorb approximately 85 to 95% of UV (Cole et al., 2016). To be effective, it is important that those UV filters be uniformly spread on skin. This requires a stable, homogeneous and non-flocculated dispersion of these UV filters.

To facilitate their dispersion, titanium dioxide and zinc oxide-are usually used with an average particle size smaller than 100 nm.

However, there are safety concerns surrounding those nanoparticles, in particular about skin penetration, risk of inhalation, eco-toxicity and bioaccumulation in the human body.

Mineral texturing agents are fine mineral powders not soluble in an oil.

Few dispersing agents can disperse those mineral powders to obtain homogeneous and stable dispersions.

In the cosmetic field, some emollients, such as C12-15 alkyl benzoate, can be used to solubilize some solid organic UV filters. However, it is more difficult for those emollients to disperse solid mineral UV filters or other ingredients in powder form.

Moreover, some cosmetic products require high contents of mineral ingredient in powder form to guarantee satisfactory coverings.

Therefore, it remains an need for a dispersing agent enabling the obtention of homogeneous and stable dispersions of particles, even with a high content of mineral ingredient in powder form.

The inventors surprisingly found that a specific compound could efficiently disperse mineral ingredients in powder form, resulting in stable homogeneous dispersions.

Accordingly, the present invention relates to a dispersion comprising or consisting of:
- a lipophobic ingredient in powder form;
- isocetyl isoarachidol;
- optionally, an emulsifier;
wherein the weight ratio lipophobic ingredient in powder form / isocetyl isoarachidol is comprised between 0.4 and 3.

In the present application, unless otherwise indicated, all ranges of values used are to be understood as being inclusive limits.

In the present application, by "dispersion", it is intended to mean a suspension of a chemical which is solid into another chemical which is liquid.

Isocetyl isoarachidol is a branched alcohol comprising 36 carbon atoms, which is known as emollient.

The lipophobic ingredient in powder form is preferably an ingredient usually used in the field of cosmetics.

In particular, the lipophobic ingredient in powder form is a mineral pigment, a mineral ultraviolet (UV) filter, an organic dye, and /or a mineral texturing agent.

Preferably, the mineral pigment is chosen among the group consisting of metal oxides, such as oxides of titanium (TiO₂), zinc (ZnO), iron (Fe₂O₃), zirconium (ZrO₂), silicon (SiO₂), manganese (MnO), aluminum (Al₂O₃), cerium (Ce₂O₃); carbonates, such as calcium carbonate and magnesium carbonate; diatomaceous earth; silicates such as mica; and mixtures thereof.

Preferably, the mineral UV filter is titanium dioxide and/or zinc oxide.

Zinc oxide and mica can be used as mineral pigments and/or as mineral texturing agents.

The mineral pigments and mineral UV filters may be surface-treated ("coated") or not. This surface treatment can consist in providing the mineral pigments and mineral mineral UV filters with a thin hydrophilic or hydrophobic inorganic and/or organic layer by methods known by the skilled person.

The inorganic surface coatings might be of silica (SiO₂), aluminium hydroxide (Al₂(OH)₃), aluminium oxide (Al₂O₃), alumina, sodium hexametaphosphate (Na(PO₃)₆), sodium metaphosphate ((NaPO₃)ₙ), silica (SiO₂), or iron oxide (Fe₂O₃).

The organic surface coatings might be of aluminum stearate, stearic acid, lauric acid, dimethylpolysiloxane, dimethicone, methylypolysiloxane, simethicone, either as single components or as mixtures.

Preferably, neither mineral pigments nor mineral UV filters are surface-treated.

An organic dye is an organic substance which may be natural or synthetic.

The dye is preferably chosen among dyes usually used in the field of cosmetics, such as red dyes Cl 15850 (Red 6 Lake) and CI 45410 (Red 28 Lake).

A mineral texturing agent is used in a cosmetic formulation to modify, adapt or adjust the physical such as the touch of finished products and visual appearance of the product's texture.

Preferably, the texturing agent is chosen among the group consisting of talc; kaolin; bentonite; powdered botanical extracts starch, such as rice starch, corn starch; mica; sericite; magnesium stearate; micronized rice powder; silica powder; silk powder; lauroyl lysine; zinc oxide; allantolin; and mixtures thereof.

The particles of the lipophobic ingredient in powder form may be micronized or non-micronized. The smaller the particle size, the easier the dispersion.

However, isocetyl isoarachidol is not only a good dispersing agent for nanoparticles, but also for larger particles.

Preferably, the lipophobic ingredient in powder form presents a particle size larger than 100 nm.

By "particle size" it is intended to mean the average particle size which is the average diameter based on the median particle sizes distribution of all particles representing 50% of the particle volume.

The particle size may be measured using any method known by the person skilled in the art. In particular, the particle size may be determined by sieve analysis, laser diffraction, dynamic light scattering, or direct imaging techniques. Preferably, the particle size is determined by laser diffraction.

Examples 1 and 2 illustrate this dispersing property, in which 40% by weight of a lipophobic ingredient in powder form with particle sizes larger than 100 nm, could be homogeneously dispersed in 60% by weight of isocetyl isoarachidol.

Preferably, the particle size of the lipophobic ingredient in powder form is of at least 500 nm, more preferably of at least 1 µm.

Moreover, all dispersions obtained with the use of isocetyl isoarachidol remained homogeneous at least 1 month at 25°C.

Indeed, the dispersions according to the invention are stable.

By "stable dispersion", it is intended to mean that the lipophobic ingredient in powder form remains homogeneously dispersed into the dispersing agent for at least one month at 25°C, with less than 5 vol% of the dispersing agent layer formation on top of the dispersion, the volume percentage being based on the volume of the dispersion.

Thus, isocetyl isoarachidol is a particularly good dispersing agent for mineral ingredients in powder form, since the resulting dispersions obtained are homogeneous dispersions of particles of a lipophobic ingredient, and are stable.

Preferably, in the dispersion according to the invention, the weight ratio lipophobic ingredient in powder form / isocetyl isoarachidol is comprised between 0.5 and 2.5, more preferably between 0.5 and 2, even more preferably between 0.54 and 1.85, such as between 0.6 and 1.85 and between 0.65 and 1.5.

Advantageously, in the dispersion according to the invention:
- the lipophobic ingredient in powder form represents at least 35% by weight;
- the isocetyl isoarachidol represents at least 35% by weight;
- the optional emulsifier represents from 0 to 5% by weight;
weight percentages being based on the weight of the dispersion.

Preferably, the quantity of the lipophobic ingredient in powder form is comprised between 35 and 65%, such as between 40 and 65%, more preferably between 40 and 60%, such as 40 and 55%, even more preferably between 40 and 50% by weight based on the weight of the dispersion.

Preferably, the quantity of isocetyl isoarachidol is comprised between 35 and 65%, such as between 35 and 60%, more preferably between 40 and 60%, such as 45 and 60%, even more preferably between 50 and 60% by weight based on the weight of the dispersion.

The present also relates to a process for preparing a dispersion according to the invention, by mixing isocetyl isoarachidol with a lipophobic ingredient in powder form.

To obtain an homogeneous dispersion, the mixing is preferably performed with a mechanical stirrer at a stirring rate of at least 600 rpm, more preferably of at least 700 rpm, even more preferably of at least 800 rpm, such as 1000 rpm.

During the mixing, it is not necessary to heat. The mixing may be performed at room temperature, such as at a temperature of 25°C +/- 5°C.

Optionally, the process may further comprise an additional step of homogenization.

When the process comprises a step of homogenization, the homogenization can be performed using a homogenizer, such as at 10000 rpm for 1 minute.

The resulting dispersions can be easily obtained through an optimized production process, requiring less energy and time.

The present invention also concerns a cosmetic product comprising the dispersion according to the invention, and a preservative, an active ingredient, and/or a perfume.

The dispersion is as described above, including preferential and advantageous features.

More particularly, the preservative is chosen among the group consisting of phenoxyethanol, glycol ethers, benzoic acid, sorbic acid, salicylic acid, glycols, parabens, thiazolinones, ethylhexylglycerin, aldehydes, and mixtures thereof.

More particularly, the active ingredient is chosen among the group consisting of allantoin, tocopherol, ascorbic acid, ascorbyl palmitate, vitamin D, polyphenols, flavonoids, and mixtures thereof.

The cosmetic product is preferably pasty to solid. In particular, it can be in the form of a paste, a stick or a powder.

The cosmetic product can be a waterless formulation or not.

When water is present in the cosmetic product, the later may be in the form of a water-in-oil or oil-in-water emulsion.

The cosmetic product can be a product for make-up such as a lipstick, eye lid decoration, blush; a sunscreen; a hair coloring product; a diaper cream.

Preferably, the quantity of the dispersion according to the invention is of at least 15%, more preferably at least 20% by weight based on the weight of the cosmetic product.

Preferably, the quantity of the dispersion according to the invention is of at most 80%, more preferably at most 60%, even more preferably at most 40% by weight based on the weight of the cosmetic product.

Preferably, the quantity of the dispersion according to the invention is comprised between 15 and 80% by weight, more preferably between 15 and 60% by weight, even more preferably between 15 and 40% by weight, such as between 20 and 40% by weight based on the weight of the cosmetic product.

The cosmetic product according to the invention may further comprise an emulsifier, an emollient, a viscosifying agent, a film-forming agent, a liquid or solid organic UV filter, a humectant and/or a solvent.

The emulsifier, the emollient, the viscosifying agent, the film-forming agent, the liquid organic UV filter, the solid organic UV filter, the humectant and the solvent can be any one among those known by the skilled person.

More particularly, the emulsifier is chosen among the group consisting of polyglycerol esters, sorbitan esters, lecithin, monoglycerides, diglycerides, and mixtures thereof.

More particularly, the emollient is chosen among the group consisting of triethylhexanoin; Helianthus Annuus (Sunflower) Seed Oil; petrolatum; mineral oils; lanolin; cocoa butter; synthetic esters such as isoamyl laurate; and mixtures thereof.

More particularly, the viscosifying agent is chosen among the group consisting of Cera Alba (beeswax), Euphorbia Cerifera (Candelilla) wax, Oryza Sativa (rice) Bran wax, myristyl myristate, butyrospermum parkii butter (shea butter), triisostearin, cetearyl alcohol, and mixtures thereof.

More particularly, the film-forming agent is chosen among the group consisting of polyvinylpyrrolidone (PVP), acrylates, acrylamides, copolymers and mixtures thereof.

More particularly, the liquid organic UV filter is chosen among the group consisting of ethylhexyl methoxycinnamate, octocrylene, octyl-dimethylaminobenzoate, p-amyl dimethyl p-amino benzoic acid (padimate-A), and mixtures thereof.

More particularly, the solid organic UV filter is chosen among the group consisting of More particularly, the solid organic UV filter is chosen among the group consisting of butyl methoxydibenzoylmethane, benzophenone-3, diethylamino hydroxybenzoyl hexyl benzoate, bis-ethylhexyloxyphenol methoxyphenyl triazine, ethylhexyl triazone, p-amino benzoic acid, and mixtures thereof.

More particularly, the humectant is chosen among the group consisting of triethylene glycol, di- or tripropylene glycol, propylene glycol, glycerin, sorbitol, hexylene and butylene glycol, urea, and mixtures thereof.

More particularly, the solvent is chosen among the group consisting of water, ethanol, and mixtures thereof.

Preferably, the cosmetic product does not contain any dimer diol and/or ethyl cellulose polymer.

A dimer diol is a mixture of cyclic and noncyclic isomers, known by the skilled person. It is usually obtained by:
- dimerization of unsaturated fatty acid(s) or ester(s) thereof followed by reduction of respectively acid or ester groups into hydroxyl groups, or
- dimerization of unsaturated fatty alcohol(s).

Preferably, unsaturated fatty acid(s) or ester(s) thereof and unsaturated fatty alcohol(s) are respectively mono carboxylic acid(s) or ester(s) and mono alcohol(s), each hydrocarbon chain of fatty acid(s) or fatty alcohol(s) comprising between 18 and 24, preferably between 18 and 22 carbon atoms. Also, dimer diol molecules preferably comprise between 36 and 48, more preferably between 36 and 44 carbon atoms.

An ethyl cellulose polymer is a derivative of cellulose, wherein some hydroxyl groups on the repeating glucose units are converted into ethoxyl groups. In particular, the ethyl cellulose polymer is of formula (I): 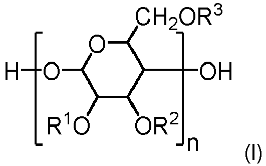 wherein
- R¹, R² and R³, identical or different, represent H or CH₂CH₃, and
- n, an integer, is of at least 2.

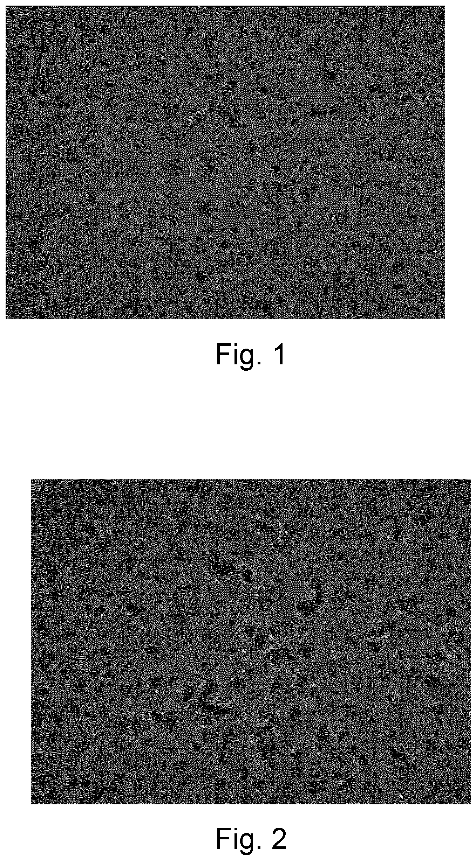

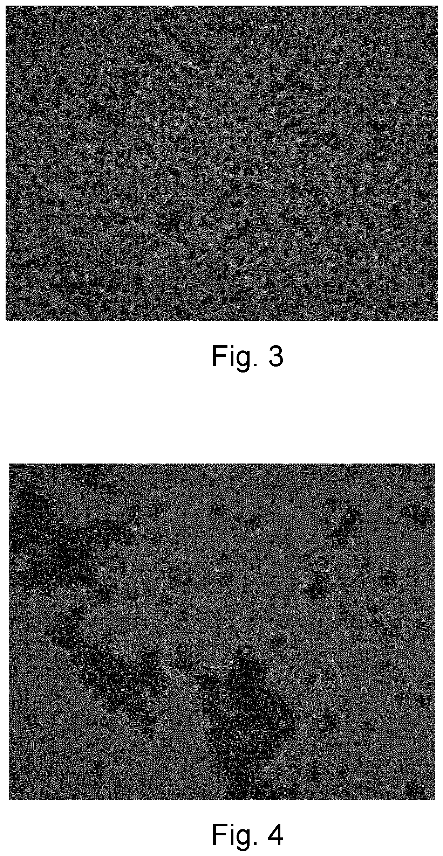

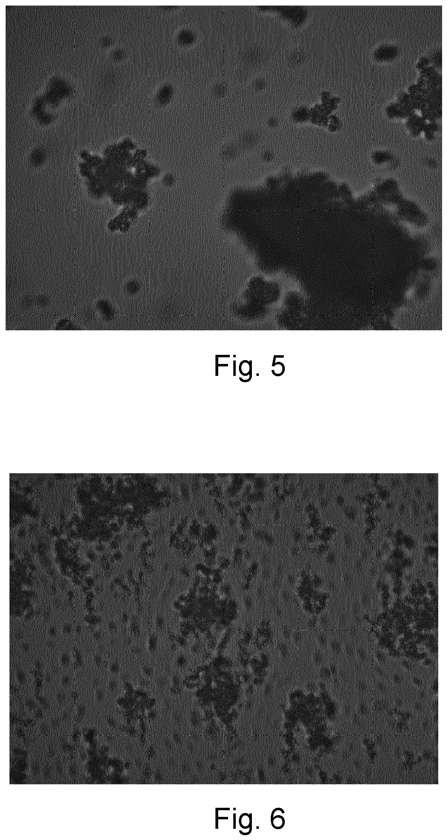

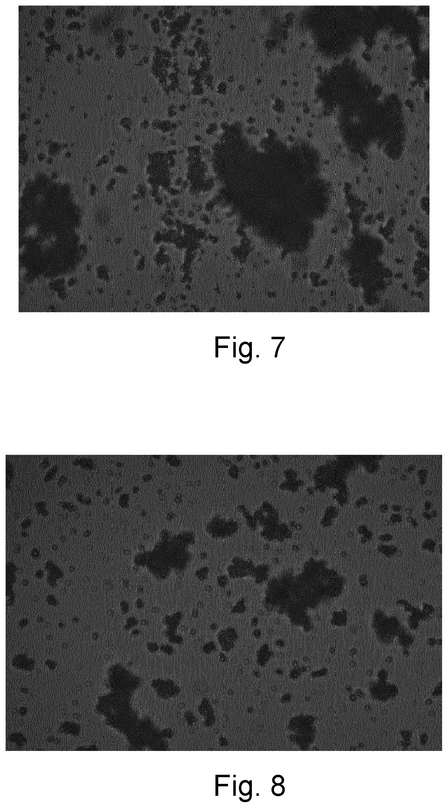

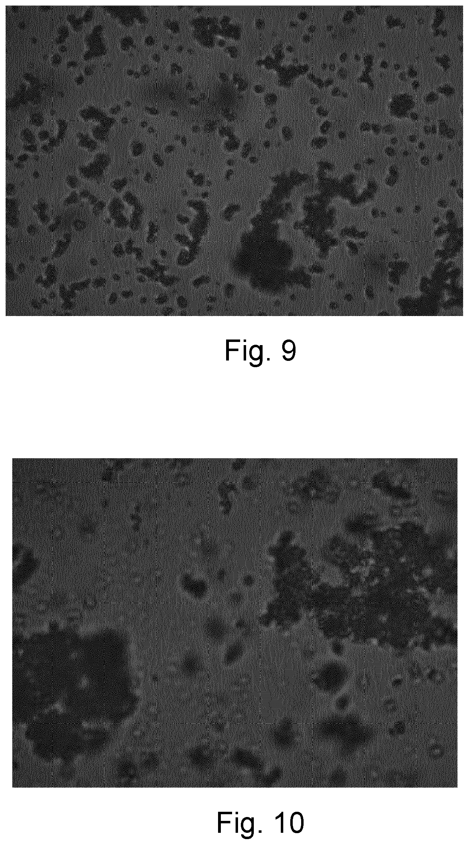

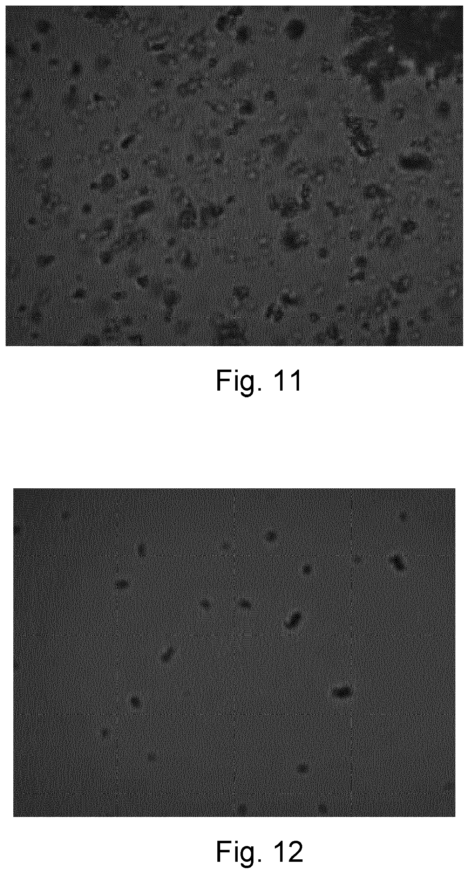

As illustrated by Figures 1-3, isocetyl isoarachidol can prevent flocculation of particles of those said mineral ingredients. On the contrary, on Figures 4-12, aggregations may be observed when an emollient other than isocetyl isoarachidol is used, or the dispersion were not stable.

The present invention also relates to an use of isocetyl isoarachidol as a dispersing agent of lipophobic ingredient in powder form.

Advantageously, in the use according to the invention, the weight ratio lipophobic ingredient in powder form / isocetyl isoarachidol is comprised between 0.4 and 3.

Preferably, in the use according to the invention, the weight ratio lipophobic ingredient in powder form / isocetyl isoarachidol is comprised between 0.5 and 2.5, more preferably between 0.5 and 2, even more preferably between 0.54 and 1.85, such as between 0.6 and 1.85 and between 0.65 and 1.5.

Advantageously, the use according to the invention, is to form a stable dispersion.

By "stable dispersion", it is intended to mean that the lipophobic ingredient in powder form remains homogeneously dispersed into the dispersing agent for at least one month at 25°C, with less than 5 vol% of the dispersing agent layer formation on top of the dispersion, the volume percentage being based on the volume of the dispersion.

Another field of application also requires the dispersion of high content of mineral pigments.

The present invention also concerns a paint comprising the dispersion according to the invention, and a drier and/or a binder.

The dispersion is as described above, including preferential and advantageous features.

The drier is preferably chosen from the driers used in the field of coatings.

Examples of driers are metal salts (such as cobalt, manganese, iron, zirconium, strontium, aluminum, calcium, barium, bismuth, zinc, lithium and potassium) of aliphatic natural or synthetic acids (such as linoleic acid, naphthenic acid, 2-ethyl-hexanoic acid and neodecanoic acid).

Preferably, the metal of the metal salt is chosen among the group consisting of cobalt, manganese, iron, zirconium, calcium, potassium, aluminum, and mixtures thereof.

Preferably, the quantity of the drier is of at least 1% by weight based on the weight of the paint.

The binder may be an alkyd resin, an epoxy resin, a polyester and vinylester resin, an acrylic resin or an polyurethane resin.

Preferably, the quantity of the binder is of at least 30% by weight based on the weight of the paint.

In the paint according to the invention, the quantity of the dispersion according to the invention is preferably of at least 20%, more preferably at least 25% by weight based on the weight of the cosmetic product.

In the paint according to the invention, the quantity of the dispersion according to the invention is preferably of at most 80%, more preferably at most 60%, even more preferably at most 40% by weight based on the weight of the cosmetic product.

Preferably, in the paint according to the invention, the quantity of the dispersion according to the invention is comprised between 20 and 80% by weight, more preferably between 20 and 60% by weight, even more preferably between 20 and 40% by weight, such as between 25 and 40% by weight based on the weight of the cosmetic product.

A preferred paint according to the invention comprises:
- 20-80% by weight of a dispersion according to the invention;
- 30-60% by weight of a binder;
- 1-3% by weight of a drier.

Preferably, the paint is a white paint.

Advantageously, in the paint according to the invention, the lipophobic ingredient in powder form in the dispersion according to the invention is titanium dioxide.

The invention is further described in the following examples, given by way of illustration, with reference to the figures:
- Figure 1 represents a picture of the dispersion of TiOz into isocetyl isoarachidol;
- Figure 2 represents a picture of the dispersion of ZnO into isocetyl isoarachidol;
- Figure 3 represents a picture of the dispersion of coated Fe₂O₃ into isocetyl isoarachidol;
- Figure 4 represents a picture of the dispersion of TiOz into C12-15 alkyl benzoate;
- Figure 5 represents a picture of the dispersion of ZnO into C12-15 alkyl benzoate;
- Figure 6 represents a picture of the dispersion of coated Fe₂O₃ into C12-15 alkyl benzoate;
- Figure 7 represents a picture of the dispersion of TiO₂ into isoamyl laurate;
- Figure 8 represents a picture of the dispersion of TiO₂ into isostearoyl alcohol;
- Figure 9 represents a picture of the dispersion of TiO₂ into octyldodecanol;
- Figures 10 represents a picture of the dispersion of ZnO into isoamyl laurate;
- Figures 11 represents a picture of the dispersion of ZnO into isostearoyl alcohol;
- Figures 12 represents a picture of the dispersion of ZnO into octyldodecanol; pictures have been obtained using the microscope Zeiss AX10 - 100X Lens x object10X = 1000X;
- Figure 13 represents diagram relating to the evolution of the dynamic viscosity of dispersions according to the shear rate applied, the dispersions comprising respectively 40 and 50 wt% of titanium dioxide in 60 and 50 wt% of isocetyl isoarachidol;
- Figure 14 represents a diagram of the intensity of Erythema of lipsticks L1 and CL1-CL2, each lipstick comprising a different emollient;
- Figure 15 represents a diagram of the melanin intensity of lipsticks L1 and CL1-CL2, each lipstick comprising a different emollient;
- Figure 16 represents diagram relating to the evolution of the viscosity of lipsticks L1 and CL1-CL2 according to the temperature.

### Chemicals used in Examples:

- Mineral pigments, mineral UV filters, lipophobic organic dyes and texturizing agent:
   - titanium dioxide, powder, -325 mesh, ≥99% trace metals, average particle size of 45 µm, basis from Sigma Aldrich;
   - zinc oxide puriss. p.a., ACS reagent, ≥99.0% (KT) from Sigma Aldrich;
   - iron oxide coated with jojoba ester, KOBO-IYJE3 from Kobo Products;

Zinc oxide and iron oxide were sieved before using them, to present an average particle size of 75 µm.

The particle size was measured using a sieve tower analyzer compliant to DIN ISO 3310-1 and ASTM E11-16 standards.
- Emollients:
   - isocetyl isoarachidol: Radiastar 1436 from Oleon;
   - C12-15 alkyl benzoate : Finsolv TN from Innospec;
   - isoamyl laurate: Jolee 7750 from Oleon;
   - isostearoyl alcohol: Radianol 1980 from Oleon;
   - octyldodecanol: Radiastar 1420 from Oleon;
   - triethylhexanoin: Radia 7610 from Oleon;
   - Helianthus Annuus (Sunflower) Seed Oil : Radia 6121 from Oleon;
- Viscosifying agents:
   - Euphorbia Cerifera (Candellila) Wax: Candelilla wax from Kerfoot
   - Oryza Sativa Bran (Rice Bran) Wax from Kerfoot;
   - Cera Alba: Beeswax organic from Kerfoot;
   - myristyl myristate: Radia 7744 from Oleon;
   - butyrospermum parkii butter (shea butter) from Kerfoot;
   - triisostearin: Radia 7373 from Oleon;
   - cetearyl alcohol: Ecorol 68/30 from Ecogreen;
- Emulsifiers:
   - glyceryl stearate (and) PEG-100 stearate: Radia 7490 from Oleon;
   - polyglycerol polyricinoleate and sorbitan isostearate: Jolee 7887 from Oleon;
- Water thickener: xanthan gum: Ziboxan F6070-Xanthan Gum Food Grade from Deosen;
- Humectant: Glycerine Radia 4811K from Oleon;
- Liquid organic UV filter: ethylhexyl methoxycinnamate: Eusolex 2292 from Merck;
- Antioxidant: tocopheryl acetate: vitamin E from BASF;
- Preservative: phenoxyethanol from Arkema;
- Flavoring agent:
   - Aroma: strawberry emulco from Green House Ingredients.

### Example 1: Preparation of dispersions

Dispersions were prepared by adding respectively 40 wt% of titanium dioxide (TiO₂), zinc oxide (ZnO) or coated iron oxide (Fe₂O₃), into 60 wt% of an emollient, then stirred still homogeneous, and finally stirred by a homogenizer at 10000 rpm for 1 minute.

The different dispersions prepared appear in Table 1 bellow.

**Table 1: Dispersions D1-D3 according to the invention and comparative dispersions CD11-CD13, CD21-CD22, CD31-CD32 and CD41-CD42**

| | Emollient (60 wt%) | Mineral pigment/ UV filter (40 wt%) | Figure |
|---|---|---|---|
| D1 | isocetyl isoarachidol | titanium dioxide | 1 |
| D2 | isocetyl isoarachidol | zinc oxide | 2 |
| D3 | isocetyl isoarachidol | iron oxide coated | 3 |
| CD11 | C12-15 alkyl benzoate | titanium dioxide | 4 |
| CD12 | C12-15 alkyl benzoate | zinc oxide | 5 |
| CD13 | C12-15 alkyl benzoate | iron oxide coated | 6 |
| CD21 | isoamyl laurate | titanium dioxide | 7 |
| CD31 | isostearoyl alcohol | titanium dioxide | 8 |
| CD41 | octyldodecanol | titanium dioxide | 9 |
| CD22 | isoamyl laurate | zinc oxide | 10 |
| CD32 | isostearoyl alcohol | zinc oxide | 11 |
| CD42 | octyldodecanol | zinc oxide | 12 |

### Example 2: Characteristics of the dispersions

### 2.1 Microscopic pictures

The quality of dispersions obtained in Example 1 was evaluated one day after their formation using a microscope Zeiss AX10 with 10X 100X magnification.

It could be observed on Fig. 1-3, as described in the presentation of the Figures, that isocetyl isoarachidol lead to homogeneous spread of small particles of mineral pigments and/or mineral UV filters (D1-D3). No flocculation was observed.

The dispersions CD11-CD13, CD21-CD22, CD31 and CD41 formed with a dispersant other than isocetyl isoarachidol, lead to a non-homogeneous spread of mineral pigments/UV filters, in particular, flocculants can be seen on Fig.4 - Fig.10, as described in the presentation of the Figures.

Dispersions CD32 and CD42 looked acceptable, since less flocculation was observed.

### 2.2 Stability

The stability of the dispersions D1-D3, CD32 and CD42 were then tested.

The dispersions D1-D3, CD32 and CD42 prepared in Example 1 were maintained 1 month at 25°C.

As shown in Table 2, after one month, the dispersions according to the invention D1-D3 were still homogeneous with only traces of the dispersing agent on top of the dispersions D1 and D2.

On the opposite, the comparative dispersions CD32 and CD42 presented respectively 5 vol% and 20 vol% of dispersing agent on top of the dispersion.

**Table 2: Stability of dispersions D1-D3 according to the invention and of comparative dispersions CD32 and CD42**

| | Separation after | |
|---|---|---|
| | 1 day | 1 month |
| D1 | traces | traces |
| D2 | 0 | traces |
| D3 | 0 | 0 |
| CD32 | traces | 5 vol% |
| CD42 | traces | 20 vol% |

The dispersions according to the invention not only disperse homogeneously the mineral powders, without formation of large flocculants, but also remain stable for at least 1 month at 25°C, without formation of 5 vol% or more of dispersing agent layer on top of the dispersion.

### 2.3 Shear-thinning property

### 2.3.1 Preparations of dispersions according to the invention

Two dispersions of titanium dioxide into isocetyl isoarachidol were prepared according to the method described in Example 1, each comprising respectively 40 and 50 wt% of titanium dioxide.

### 2.3.2 Measurement of the dynamic viscosity

Dynamic viscosities of each dispersions according to the invention were measured using a Rheometer DHR, controlled stress, TA instruments, plate/plate geometry, plate 4cm diameter, Gap: 250 micrometer; at 25°C, 24 hours after their preparation.

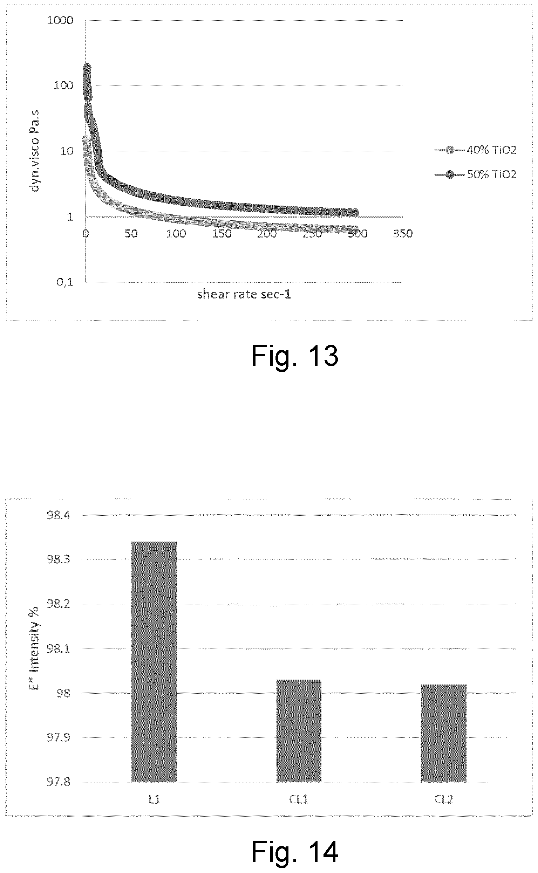

On Fig. 13, it can be observed that the viscosity of all the dispersions decreases when the shear rate increases. Dispersions according to the invention comprising a high content of mineral pigment/UV filter such as 40 wt% and 50wt% of titanium dioxide are shear-thinning. It means that the mineral pigment/UV filter particles contained in the dispersion according to the invention can be spread easily on the skin.

### Example 3: Cosmetic products

### 3.1 Lipstick

The lipsticks were prepared by using ingredients and quantities described in Table 3.

Phase A was prepared by heating the corresponding ingredients at 80°C until melted.

Phase B was prepared by mixing the corresponding pigments into the dispersing agent until obtaining a paste using a triple roll mill.

Phase B was heated to a temperature of 60°C.

Phase B was poured into phase A at 60°C.

Phase C was then added, at a temperature of at most 40°C.

Then the mixture was placed in the fridge at 4°C to solidify.

**Table 3: Formulation of lipstick L1 according to the invention and of comparative lipsticks CL1-CL2**

| | INCI names | Function | Lipstick L1 (wt%) | Comparative lipstick CL1 (wt%) | Comparative lipstick CL2 (wt%) |
|---|---|---|---|---|---|
| A | Euphorbia Cerifera (Candelilla) Wax | viscosifying agent | 17.0 | 17.0 | 17.0 |
| | Oryza Sativa (Rice) Bran Wax | viscosifying agent | 16.0 | 16.0 | 16.0 |
| | Cera Alba (Beeswax) | viscosifying agent | 5.0 | 5.0 | 5.0 |
| | Myristyl Myristate | viscosifying agent | 5.0 | 5.0 | 5.0 |
| | Butyrospermum Parkii (Shea) Butter | viscosifying agent | 3.0 | 3.0 | 3.0 |
| | Triethylhexanoin | emollient | 13.0 | 13.0 | 13.0 |
| | Helianthus Annuus (Sunflower) Seed Oil | emollient | 13.9 | 13.9 | 13.9 |
| | Cetyl Alcohol | viscosifying agent | 5.0 | 5.0 | 5.0 |
| B | Isocetyl Isoarachidol | emollient/dispersing agent | 12.0 | | |
| | Octyldodecanol | emollient/dispersing agent | | 12.0 | |
| | C12-C15 Alkyl Benzoate | emollient/dispersing agent | | | 12.0 |
| | Cl77891 (Titanium Oxide) | mineral pigment | 1.8 | 1.8 | 1.8 |
| | Cl15850 (Red 6 Lake) | lipophobic organic dye | 2.6 | 2.6 | 2.6 |
| | Cl45410 (Red 28 Lake) | lipophobic organic dye | 2.6 | 2.6 | 2.6 |
| | Cl77491 (Iron Oxide) (and) Jojoba Esters | mineral pigment | 1.0 | 1.0 | 1.0 |
| C | Ethylhexyl Methoxycinnamate | organic UV filter | 1.4 | 1.4 | 1.4 |
| | Tocopheryl Acetate | antioxidant | 0.2 | 0.2 | 0.2 |
| | Aroma | flavoring agent | 0.5 | 0.5 | 0.5 |

### 3.1.1 Color intensity

To evaluate the color intensity of the lipsticks, measurements of redness or Erythema intensity (E*) were made using a Mexameter MX18 probe, which detects redness via absorption/reflection, at wavelengths of 568 nm and 660 nm.
Units: arbitrary Mexameter^{®} units (0-999 for erythema);
Measurement time: 1 s, Accuracy: ± 5%.

As shown on Figure 14, the lipstick L1 according to the invention comprising isocetyl isoarachidol as dispersing agent, presents the highest color intensity as it gives the highest value of Erythema intensity, compare to comparative lipsticks CL1 and CL2 comprising respectively octyldodecanol and C12-C15 alkyl benzoate as dispersing agent.

### 3.1.2 Coverage

To evaluate the coverage of the lipsticks, measurements of melanin intensity (M*) were made using a Mexameter MX18 probe, which detects via absorption/reflection the melanin intesity at wavelengths of 568 nm (Green), 660 nm (Red) and 870nm (Infrared).
Units: arbitrary Mexameter^{®} units (0-999 for melanin);
Measurement time: 1 s, Accuracy: ± 5%.

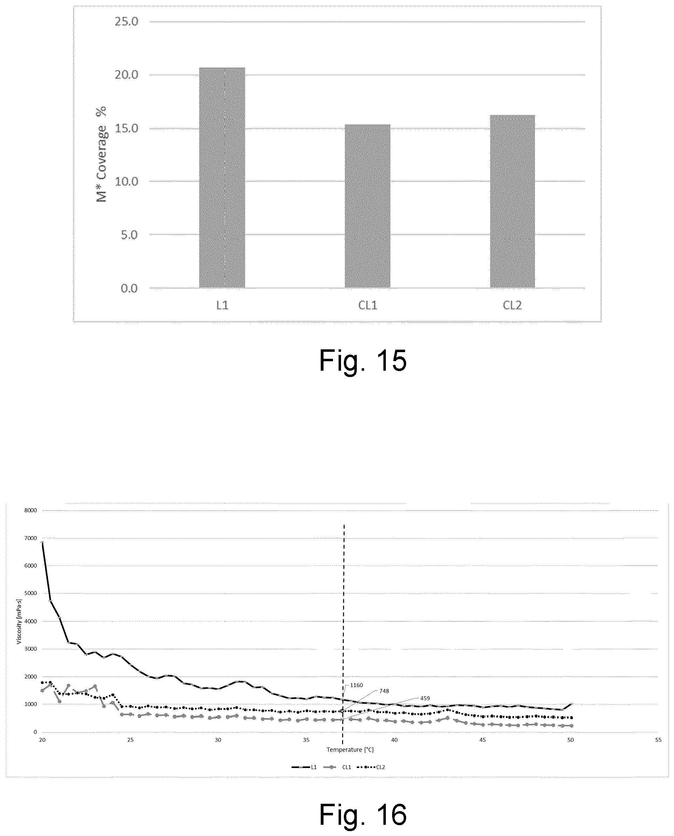

As shown on Figure 15, the lipstick L1 according to the invention comprising isocetyl isoarachidol as dispersing agent, presents the highest coverage as it gives the highest value of Melanin (M*), compare to comparative lipsticks CL1 and CL2 comprising respectively octyldodecanol and C12-C15 alkyl benzoate as dispersing agent.

### 3.1.3 Rheology

The pigment suspension's rheological characteristics play a significant role in determining how it will behave both during storage and use.

To avoid melting during storage of lipstick, it is necessary for the viscosity to be adequate while no shear is applied to the lipstick.

And for a good spread on lips, a shear-thinning property is necessary.

The measurements of dynamic viscosities were taken using a rheometer MCR302 from Anton Paar with a cone-plate CP25. The cone parameters were as follows: CP-25 mobile (Diameter 24.798 mm, Angle of Cone = 1.982°; Truncate = 103 µm), temperature control unit P-PTD200.

### Method Oscillatory Temperature Sweep mode (20-50°C)

As it can be seen on Figure 16, the lipstick L1, at 20°C, has the highest dynamic viscosity (6854.7 mPa·s) and at the optimum temperature of lipstick application (37°C, the body temperature), a dynamic viscosity of 1160 mPa s.

It means that the lipstick according to the invention presents a better ability to retain the shape of the lipstick. It also exhibits a shear thinning property since the dynamic viscosity decreases steadily with increasing temperature.

### 3.2 Sunscreen

The sunscreen according to the invention was prepared by using ingredients according to the quantities described in Table 4.

Phase A ( water phase) was prepared by sprinkling the xanthan gum on water and glycerin under high stirring until a gel formation. Then, phase A was heated to 80°C.

Phase B (oil phase) was prepared by mixing the corresponding ingredients. Phase B was also heated to 80°C.

Phase B was poured into phase A.

The oil-in-water emulsion was then cooled to 40°C.

Phase C was added into the emulsion, at a temperature of at most 40°C.

If needed, the pH is corrected to be between 4.5 and 6.

**Table 4: Formulation of a sunscreen according to the invention**

| | INCI names | Function | %w/w |
|---|---|---|---|
| A | Aqua | solvent | 71 |
| | Glycerin | humectant | 3 |
| | Xanthan gum | water thickener | 0.5 |
| B | Glyceryl Stearate (and) PEG-100 Stearate | emulsifier | 4 |
| | Isocetyl Isoarachidol | emollient/dispersing agent | 10 |
| | Titanium Dioxide (and) Silica | mineral UV filter | 10 |
| C | Phenoxyethanol (and) Ethylhexylglycerin | preservative | 1 |
| | Tocopheryl Acetate | antioxidant | 0.5 |

### 3.3 Diaper cream

The baby cream was prepared by using ingredients and quantities described in Table 5.

Phase A was prepared by mixing the mineral pigment and texturizing agent with the dispersing agent and adding the emulsifier, then the viscosifying agents.

Phase A is heated at 80°C until melted.

Phase B was prepared by mixing the different ingredients.

Phase B was heated at 80°C.

Phase B was poured into phase A at 80°C.

Phase C was then added, at a temperature of at most 40°C.

**Table 5: Formulation of a diaper cream according to the invention**

| | INCI names | Function | %w/w |
|---|---|---|---|
| A | Polyglycerol polyricinoleate and sorbitan isostearate | emulsifier | 3.5 |
| | triisostearin | viscosifying agent | 0.5 |
| | Isocetyl isoarachidol | emollient/dispersing agent | 15 |
| | Cetearyl alcohol | viscosifying agent | 1 |
| | Zinc oxide | mineral pigment and texturing agent | 10 |
| B | Glycerin | humectant | 4 |
| | Magnesium sulfate | osmotic pressure regulator | 0.8 |
| | aqua | solvent | 64.2 |
| C | Phenoxyethanol (and) Ethylhexylglycerin | preservative | 1.0 |

## Claims

1. Dispersion comprising or consisting of:
- a lipophobic ingredient in powder form;
- isocetyl isoarachidol;
- optionally, an emulsifier;
wherein the weight ratio lipophobic ingredient in powder form / isocetyl isoarachidol is comprised between 0.4 and 3.

2. Dispersion according to claim 1, wherein:
- the lipophobic ingredient in powder form represents at least 35% by weight;
- the isocetyl isoarachidol represents at least 35% by weight;
- the optional emulsifier represents from 0 to 5% by weight;
weight percentages being based on the weight of the dispersion.

3. Process for preparing a dispersion according to claim 1 or 2, by mixing isocetyl isoarachidol with a lipophobic ingredient in powder form.

4. Cosmetic product comprising the dispersion according to claim 1 or 2, and a preservative, an active ingredient, and/or a perfume.

5. Use of isocetyl isoarachidol as a dispersing agent of lipophobic ingredient in powder form.

6. Use according to claim 5, wherein the weight ratio lipophobic ingredient in powder form / isocetyl isoarachidol is comprised between 0.4 and 3.

7. Use according to claim 5 or 6, to form a stable dispersion.

8. Paint comprising the dispersion according to claim 1 or 2, and a drier and/or a binder.

9. Paint according to claim 8, wherein the lipophobic ingredient in powder form in the dispersion according to claim 1 or 2 is titanium dioxide.
